# EUROPEAN PATENT APPLICATION

(11) **EP 0 701 123 A1**
(43) Date of publication of application: **13.03.1996**
(21) Application number: 95305835.1
(22) Date of filing: 22.08.1995
(51) Int. Cl.: G01N 27/12, G01N 27/16

(54) **Formaldehyde vapour detector**

(30) Priority: 10.09.1994 GB 9418292
(71) Applicant: UNITED KINGDOM ATOMIC ENERGY AUTHORITY, Didcot, Oxfordshire OX11 0RA (GB)
(72) Inventor: Peat, Robert, Longcot, Oxfordshire SN7 7SU (GB)
(74) Representative: Wood, Paul Austin

(57) **Abstract**

A method of detecting formaldehyde vapour using a semiconducting oxide detector in which the sensing medium is zinc or tin oxide. An apparatus for carrying out the method also is disclosed.

## Description

The present invention relates to the detection of formaldehyde vapour.

Solid state sensors for gases are well-known. See for example, GB patent specification 2 119 933 B, 2 186 090 B, 2 186 091 B, 2 202 948 A, 2 218 523 A, 2 256 055 A, and US Patent specifications 4 880 519, 4 900 405, 4 913 792.

Conventionally, such sensors consist of a body of a material the electrical properties of which vary in the presence of one or more gases the presence of which is to be detected by the sensor, at least a pair of electrodes attached to the body of material, and means for measuring the resistance between the electrodes or potential differences appearing across them in the presence of the said gases.

The present invention relates to the detection of formaldehyde vapour by means of a detector including a gas sensor of the above type which is sensitive, specifically, to the presence in its environment of formaldehyde.

According to the present invention in one aspect, there is provided a method for the detection of gaseous formaldehyde comprising the operations of bringing a gaseous medium in which the presence of formaldehyde is to be detected into contact with a sensing element including a body of zinc oxide or tin oxide sensing medium having a pair of electrodes thereon, measuring the resistance between the electrodes, and detecting changes in the value of the resistance between the electrodes due to the presence of the gaseous formaldehyde.

Also according to the invention there is provided an apparatus for the detection of gaseous formaldehyde comprising a sensing element including a body of zinc oxide or tin oxide sensing medium having a pair of electrodes thereon, means for bringing into contact with the sensing medium a gaseous medium the presence in which of formaldehyde is to be detected, means for measuring the resistance between the electrode and means for deriving therefrom a measure of the concentration of formaldehyde in the gaseous medium.

Preferably there is included means for providing an indication of the concentration of formaldehyde in the gaseous medium. The said means for providing an indication of the concentration of formaldehyde in the gaseous medium may comprise an audio/visual alarm if a pre-determined threshold concentration is exceeded.

Preferably, the surface of the body of zinc oxide or tin oxide is platinised.

More than one pair of electrodes may be provided on the body of sensing medium, the separation between the electrodes of each pair of electrodes being different.

The invention will now be described, by way of example, with reference to the accompanying drawings, in which
Figure 1 is a representation of an apparatus embodying the invention;
Figure 2 is a flow diagram of a process for manufacturing a sensing element incorporated in the apparatus of Figure 1,
Figure 3 shows the response to formaldehyde vapour at different temperatures of a sensing element such as that produced by the process of Figure 2 in which the sensitive medium is zinc oxide, and
Figure 4 shows the response to formaldehyde vapour at different concentrations but at a constant temperature of a sensing element such as that produced by the process of Figure 2 in which the sensitive medium also is zinc oxide.

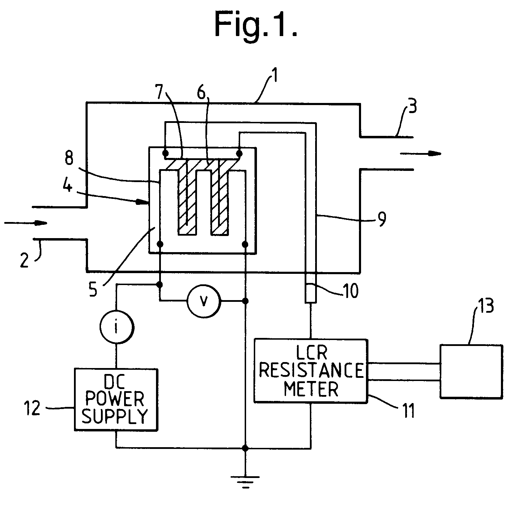

Referring to Figure 1, an apparatus for detecting formaldehyde vapour comprises a chamber 1 having an inlet 2 and an outlet 3 by means of which a gaseous medium which is to be monitored for the presence of formaldehyde vapour can be caused to flow through the chamber 1. Mounted within the chamber 1 is a sensing element 4 which consists of an alumina substrate 5 upon one side of which is deposited a sensitive medium 6 consisting of a layer of zinc oxide, and two electrodes, 7 and 8 to which contact leads 9 and 10 respectively, are attached. The leads 9 and 10 connect the sensing element 4 to a resistance measuring circuit 11. The electrodes 7 and 8 are elongated, and one of them, electrode 8, is used also as a heating element. A power supply 12 supplies power to the heating element constituted by the electrode 8. The temperature of the sensing element 4 is determined either by measuring the resistance of the electrode 8, or directly, by means of a thermocouple. The resistance measuring circuit 11 includes a meter 13, which by means of a calibration process is adapted to indicate the concentration of formaldehyde vapour in the gaseous medium under test. In addition, or alternatively, an audio/visual warning device may be provided to provide an indication that a pre-determined concentration of formaldehyde in the gaseous medium has been exceeded.

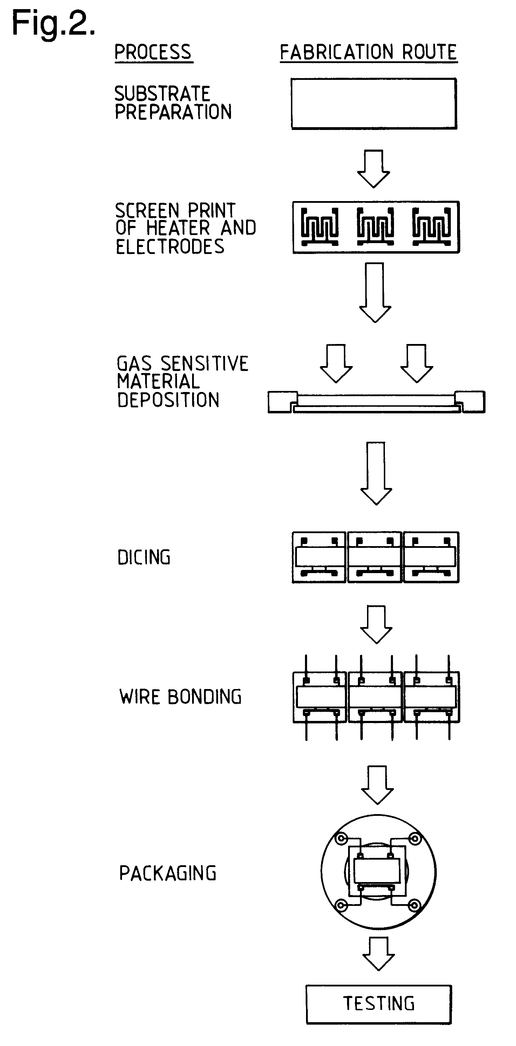

Referring to Figure 2, an alumina substrate 5 two inches square is cleaned by boiling in water for an hour, rinsed in acetone several times and then allowed to dry. A hundred sensing elements are then screen printed onto the substrate 5 or a 10 x 10 array using a platinum ink. The ink is dried at 150°C and annealed at 950°C for twenty minutes. Each sensing element consists of a pair of interdigitated electrodes 7 and 8 between which is a sensitive medium 6. The sensitive medium 6 is formed by sputtering zinc oxide through a suitable mask (not shown) onto the surface of the substrate 5 between the electrodes 7 and 8. The substrate 5 is then diced to supply a hundred separate sensing elements and platinum wires are attached to the electrodes 7 and 8 of each separate sensing element by means of silver ink. The silver ink is dried at 150°C and annealed at 750°C for three minutes, following which each sensing element is mounted onto a standard transistor header by means of spot welding.

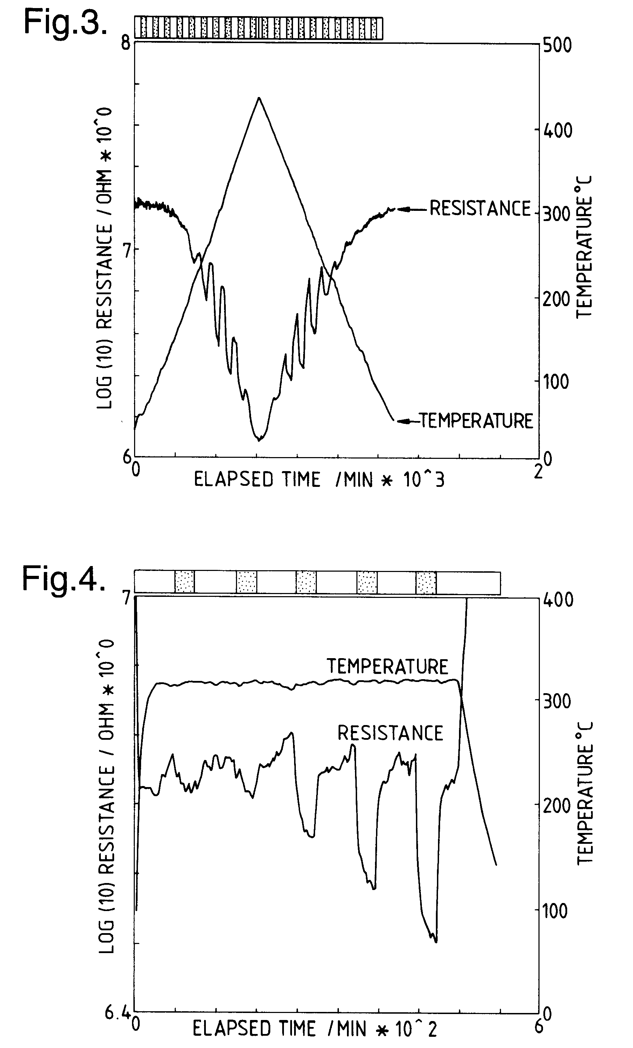

Figure 3 shows the response of a sensing element, manufactured as above, to alternate pulses of air and formaldehyde vapour/air with the formaldehyde concentration being approximately 50 ppm. The pulses of formaldehyde/air are indicated by the shaded regions at the top of the Figure. It can be seen that the sensitivity of the sensor is dependent upon its temperature with a maximum value at a temperature of approximately 315°C and a useful range of approximately 200 - 350°C.

The variation of the resistance of the same sensing element to pulses of air/formaldehyde and air at a constant temperature of 315°C are shown in Figure 4. Again the air/formaldehyde vapour pulses are shown as shaded regions at the top of the Figure. The formaldehyde vapour concentrations are, 0, 0.98, 4.7, 10.8 and 22.6 ppm, respectively. It can be seen that, over the above range of concentrations, at least, the variations in resistance of the sensing element are related to the concentration of formaldehyde vapour.

The slight response shown to nominally pure air obviously is an artefact of the test procedure and probably is due to a measure of adsorption and desorption from the walls of the apparatus. In practice, compensation for this effect could be part of a calibration procedure by means of which the meter 13 in the apparatus of Figure 1 is made to indicate directly the concentration of formaldehyde in the gaseous medium under test.

Another material which shows a similar variation in resistance upon exposure to formaldehyde vapour is tin oxide. In both cases the response can be enhanced by platinising the metal oxide material.

The invention also can be used in a manner analogous to a conventional smoke detector or alarm, in which case the chamber 1 and its associated inlet 2 and outlet 3 are omitted.

As with other forms of gas sensors in which a metal oxide semiconducting material is used as the sensing element, electrode configurations which utilise more than two electrodes can be used. For example, three electrodes may be used, the central electrode being positioned assymetrically with respect to the others. By using this electrode as a common electrode, two electrode systems with different separations between the electrodes of each pair are formed. By determining the relationship between the measured resistances between each pair of electrodes it is possible to eliminate external effects which affect each pair of electrodes equally.

## Claims

1. A method for the detection of formaldehyde comprising the operations of bringing a gaseous medium in which the presence of formaldehyde is to be detected into contact with a sensing element (4) including a body (6) of zinc oxide or tin oxide sensing medium having a pair of electrodes (7, 8) thereon, measuring the resistance between the electrodes (7, 8), and detecting changes in the value of the resistance between the electrodes (7, 8) due to the presence of the formaldehyde in the gaseous medium.

2. A method according to Claim 1 wherein the sensing medium (6) is heated to a temperature in the range 200°C to 350°C.

3. A method according to Claim 2 wherein the sensing medium (6) is maintained at a temperature of approximately 315°C.

4. An apparatus for the detection of formaldehyde comprising a sensing element (4) including a body (6) of zinc oxide or tin oxide sensing medium having a pair of electrodes (7, 8) thereon, means (1, 2, 3) for bringing into contact with the sensing medium (6) a gaseous medium the presence in which of formaldehyde is to be detected, means (11) for measuring the resistance between the electrodes (7, 8) and means (13) for deriving therefrom a measure of the concentration of formaldehyde in the gaseous medium.

5. An apparatus according to Claim 4 wherein the sensing medium (6) incorporates platinum.

6. An apparatus according to Claim 4 or Claim 5 including means (8, 12) for heating the sensing medium (6) to a pre-determined operating temperature.

7. Apparatus according to Claim 6 wherein one of the electrodes (7, 8) is adapted to act as a heating element.

8. Apparatus according to either Claim 6 or Claim 7 wherein one of the electrodes (7, 8) is adapted to act as a resistance thermometer.

9. Apparatus according to Claim 8 wherein the electrode (8) which is adapted to act as a resistance thermometer is that which is adapted to act as a heating element.

10. A method of manufacturing a sensing element for use in an apparatus according to any of Claims 4 to 9 including the operations of depositing upon an insulating substrate (5) a pair of electrodes (7, 8) and subsequently depositing upon the substrate (5) and electrodes (7, 8) a layer of a sensing medium (6) comprising zinc oxide or tin oxide.

11. A method according to Claim 10 inclduing the operation of depositing a layer of platinum upon the layer of sensing medium (6).
